# EUROPEAN PATENT SPECIFICATION

(11) **EP 3 840 640 B1**
(45) Date of publication and mention of the grant of the patent: **08.07.2026**
(21) Application number: 19758968.2
(22) Date of filing: 23.08.2019
(51) Int. Cl.: A61B 5/11, A61B 5/024, A61B 5/08, A61B 5/0205, A61B 5/113, A61B 7/00, A47C 21/00, A47C 31/00, G16H 20/30, G16H 40/63, G16H 50/20

(54) **A SLEEP MONITORING SYSTEM FOR MONITORING AND MEASURING SLEEPING ACTIVITY**
SCHLAFÜBERWACHUNGSSYSTEM ZUR ÜBERWACHUNG UND MESSUNG DER SCHLAFAKTIVITÄT
SYSTÈME DE SURVEILLANCE DU SOMMEIL POUR SURVEILLER ET MESURER L'ACTIVITÉ DU SOMMEIL

(30) Priority: 23.08.2018 GB 201813715
(43) Date of publication of application: 30.06.2021
(73) Proprietor: Marexa Oü, 75312 Rae municipality (EE)
(72) Inventor: TÄTTE, Tanel, 51011 Tartu (EE); TÄTTE, Tauri, 61708 Kambja val (EE); KONDRATIEV, Vladimir, Leningradskaya obl., 188300 (RU); VARJUND, Toomas, 75117 Kose vald (EE); ISPERT, Egert, 71109 Viljan di vald (EE); KELP, Glen, 65609 Vöru (EE); LUMISTE, Alivar, 71063 Viljandi vald (EE)
(74) Representative: Marks & Clerk LLP
(86) International application number: PCT/EP2019/072645
(87) International publication number: WO 2020/039096

(56) References cited:
- JP-A- 2006 014 813
- JP-A- 2014 023 571
- JP-A- 2017 080 636
- US-A1- 2018 195 920
- US-A1- 2019 142 348

## Description

### TECHNICAL FIELD

The present invention relates to systems for use in monitoring one or more physiological parameters of a user. More specifically, embodiments relate to the monitoring of sleep level using signal collectors and sensors that may be integrated into a mattress or a seat to detect mechanical vibrations.

### BACKGROUND

Sleep quality is of great importance to people's daily activities. Research performed by the National Sleep Foundation (USA) suggests that poor or insufficient sleep affects live of almost 45% of Americans. Many visit doctors regularly to seek improvements to the quality of their sleep. The research estimates that around 33% of the population rate their sleep as no better than fair or poor.

While it is clear that humans require sleep to function properly, the quality and quantity of sleep required is a complex problem. A great deal of research in recent years has focused on understanding sleep and its physiological and psychological effects. For example, some individuals, who sleep too little, may feel tired or fatigued during the day while others who sleep too many hours have a similar feeling of grogginess as a result of sleeping too much (see US. Patent Application Number 62108149).

Researchers continue to study many different physiological conditions during sleep to understand the complex interplay between sleep and wakeful wellbeing. To study this, different technologies have been suggested for monitoring sleep.

The monitoring of sleep is important for many reasons. Most of all, it provides actual data about sleeping - length of sleep, depth of sleep, times users go to sleep and when they wake up. Having this data in hand, people with sleep problems are motivated (or may be prompted) to visit a sleep specialist to seek treatment.

Physiological parameters, such as heart rate, can be measured using wearable electronics; however, this can be uncomfortable for the user to sleep in, thereby negatively affecting the user's quality of sleep.

US 2018/0195920 A1 discloses a pressure sensing device including an elastic tube defining a chamber and an opening in one or each of two opposite ends thereof in communication with the chamber, and a pressure sensor component sealed in the opening in one or each end of the elastic tube for detecting a change in air pressure in the chamber when the elastic tube is compressed by an external pressure.

JP 2014/023571 A discloses an alarming apparatus including: a detecting section for outputting a body movement signal by detecting the body movement of a sleeping person; a signal processing section for extracting a judgment index from the body movement signal and extracting a coarse movement signal resulting from the coarse movement of the sleeping person from the body movement signal; and a notification section for determining whether or not the sleeping person has entered rapid eye movement sleep on the basis of the judgment index and determining whether or not the sleeping person is in an awakening state on the basis of the coarse movement signal.

JP 2017/080636 A discloses a biological information collecting device for collecting biological information of a subject including: an acted object which has an internal space which can hold a prescribed fluid, and where an internal pressure varies by external actions from the subject; and a pressure detecting part for detecting variations of the internal pressure.

JP 2006/014813 A discloses a sleep diary recording apparatus provided with a bioinformation detecting part detecting bioinformation of an organism, a sleep information determination part determining sleep information based on the bioinformation, a sleep diary recording part recording the sleep diary based on the determined sleep information, and a display part displaying the recorded sleep diary.

In light of the above, there is a need for an improved means of monitoring sleep that is accurate and has improved comfort for the user.

### SUMMARY OF THE INVENTION

According to the invention there is provided a system for use in monitoring one or more physiological parameters according to claim 1, advantageous embodiments being specified in dependent claims 2-12.

### SUMMARY OF THE DISCLOSURE

According to a first aspect there is provided a system for use in monitoring one or more physiological parameters of a user. The system comprises: at least one sensing unit comprising at vibration sensors including a microphone configured to generate a microphone signal based on detected vibrations and a pressure sensor configured to generate a pressure sensor signal based on the detected vibrations; and at least one elastic tube configured to be embedded within a cushioning layer for supporting at least a portion of the user. One end of the at least one elastic tube is sealed and the other end of the at least one elastic tube is closed by the at least one sensing unit so as to form a volume that is filled with fluid and that is defined by one or more inner walls of the tube and a surface of the at least one sensing unit. The at least one elastic tube is configured to transmit vibrations from one or more sections of the at least one elastic tube to the at least one sensing unit for detection by the at least one vibration sensor. The system further comprises a controller configured to determine a body position of the user based on phase differences in the microphone and pressure sensor signals.

By providing an elastic tube for transferring vibrations, the sensing unit may be located away from the user's body thereby avoiding damage to the sensing unit by mechanical strain in the cushioning layer and also avoiding the sensing unit impacting the comfort of the cushioning layer. By making the tube from an elastic material, mechanical vibrations are more effectively transferred, whilst also allowing the tube to return to its original shape once pressure has been released to allow the tube to continue functioning.

The tube need not be cylindrical, but may be any elongate hollow structure. The tube may be straight or may comprise one or more bends along its length. The fluid may be a gas or a liquid. The fluid might be air or any other gas capable to transmitting vibrations. The tube may be made from elastic material such as rubber (e.g. natural rubber, synthetic rubber, silicone rubber, etc.), urethane, or polydimethylsiloxane.

According to an embodiment, the at least one vibration sensor comprises one or more of a microphone and a pressure sensor. The at least one vibration sensor may operate at a frequency capture range from 0 - 200 Hz. That is, the microphone and/or the pressure sensor may operate at a frequency capture range from 0 - 200 Hz.

According to an embodiment, the at least one elastic tube has a uniform cross-section along its length. This helps to avoid reflections within the tube.

According to an embodiment, the at least one elastic tube comprises one or more indents along the one or more inner walls of the tube to help prevent the tube sticking shut when crushed. The one or more indents may be in the form of one or more ridges running along the length of the tube. The one or more indents may comprise a plurality of evenly spaced indents around the circumference of the tube. This helps to prevent sticking even when crushed from a variety of angles.

According to an embodiment, the at least one elastic tube comprises a plurality of elastic tubes that are connected to a common node to form a single volume for transmitting vibrations back to the sensing unit. This allows a larger volume of the cushioning layer to be monitored.

According to an embodiment, the system further comprises a controller configured to determine one or more of: one or more physiological parameters of the user from detected vibrations; and one or more physiological states of the user from detected vibrations.

In one embodiment, the controller forms part of an external device and wherein the at least one sensing unit is configured to send data relating to the detected vibrations to the external device for processing by the controller. This might be a computer, a server, a mobile device, or any form of device with processing capabilities.

In an alternative embodiment, the controller forms part of the sensing unit.

The at least one vibration sensor may be configured to generate a vibration signal (a data transmission, such as an electrical signal, indicative of the amplitude of the detected vibrations) and send this vibration signal to the controller. This vibration signal may be analogue or digital. Where the controller forms part of an external device, the sensing unit might transmit this vibration signal to the controller, or might process the vibration signal further (e.g. via compression) before transmitting the processed signal to the controller.

According to an embodiment, the controller is configured to determine, based on the detected vibrations, one or more of a heart rate of the user, a respiration rate of the user, a body position of the user and movement of the user.

According to one embodiment, the controller is configured to determine the heart rate of the user. Determining the heart rate may comprise: filtering the detected vibrations to form filtered vibration data over a predefined frequency range; detecting local maxima in the filtered vibration data; and determining the heart rate based on the detected local maxima. The predefined frequency range may be a range that maintains a large proportion of the heartbeat information whilst filtering out noise and respiration rate information. In one embodiment, the predefined frequency range is 10 to 30 Hz.

According to one embodiment, the controller is configured to determine the respiration rate of the user, wherein determining the respiration rate comprises: detecting local maxima in the detected vibrations; and determining the respiratory rate based on the detected local maxima. The respiratory rate may be determined over different frequency range to the heart rate (e.g. over a range of 0-200 Hz rather than 10-30Hz)

According to an embodiment, the controller is configured to determine movement of the user based on the detected vibrations, wherein determining movement of the user comprises determining whether an amplitude of the detected vibrations exceeds a predefined amplitude threshold.

According to an embodiment, the at least one vibrations sensor comprises a microphone configured to generate a microphone signal based on the detected vibrations and a pressure sensor configured to generate a pressure sensor signal based on the detected vibrations. The controller is configured to determine the body position of the user, wherein the body position of the user is determined based on phase differences in the microphone and pressure sensor signals.

The determined body position might indicate whether the person is: on their back, on their stomach, on their left or on their right side.

According to one embodiment, the controller is further configured to determine the physiological state (e.g. respiration rate and/or sleep level) of the user based on specific repetitive noises within the detected vibration (e.g. within a detected respiration cycle).

According to an embodiment, the system is configured to export or store data relating to the detected vibrations (e.g. a raw vibration signal) only when certain conditions are met. These conditions may include one or more of:
(1) the sleep state of the user (e.g. the user being in deep sleep);
(2) no movements being detected within in a predefined preceding period of time (e.g. the last five minutes);
(3) the variability of the vibrations within a predefined preceding period of time (e.g. a moving average root-mean-square (RMS) value of the signal has not changed by more than a predefined amount over a predetermined period of time (e.g. 20% in the last five minutes)); and
(4) the system has detected a heartbeat or a breathing cycle (e.g. heart rate and breathing rate are able to be determined from the vibration signal).

According to an embodiment, the controller is configured to determine a sleep state of the user based on one or more of the heart rate of the user, the respiration rate of the user and movement of the user. Sleep state may be one of awake, light sleep, deep sleep and rapid eye movement (REM) sleep.

According to an embodiment, the controller is configured to perform one or more of the following: determine that the user is awake in response to determining that one or both of the heart rate and respiration rate are above a corresponding first threshold and variation in one or both of the heart rate and respiration rate is below a corresponding variation threshold; determine that the user is in rapid eye movement, hereinafter referred to as REM, sleep in response to determining that one or both of the heart rate and respiration rate are above the corresponding first threshold and the variation in one or both of the heart rate and respiration rate is above the corresponding variation threshold; determine that the user is in light sleep in response to determining that one or both of the heart rate and respiration rate are below the corresponding first threshold and above a corresponding second threshold that is less than the corresponding first threshold; and determine that the user is in deep sleep in response to determining that one or both of the heart rate and respiration rate are below the corresponding second threshold.

A corresponding first/second threshold means different first thresholds or different second thresholds may be set for heartrate and respiration rate.

According to an embodiment, the system further comprises an alarm system that is configured to issue an alarm in response to a determination that a predefined sleep state or predefined change in sleep state has been reached during an alarm period between a predefined start alarm time and a predefined end alarm time. The alarm system may be integrated within the sensing unit (i.e. in the form of a controller in the sensing unit) or may be an external device to the sensing unit (that need not be the same external device as discussed above with regard to the determination to physiological parameters).

According to an embodiment, the alarm system is configured to perform one or more of the following: issue the alarm in response to a determination that the user has transitioned from REM sleep to light sleep during the alarm period; issue the alarm in response to a determination that a current time is within a predefined period from an end alarm time and the user is in light sleep during the alarm period, the predefined period being shorter than the alarm period; and issue an alarm in response to the end alarm time being reached and the predefined sleep state or predefined change in sleep state has not been detected during the alarm period.

According to a further aspect there is provided a computer system comprising one or more processors configured to receive vibration signals indicative of vibrations detected within a cushioning layer for supporting at least a portion of the user. The vibration signals include a microphone signal sensed by a microphone a pressure sensor signal sensed by a pressure sensor. The one or more processors are further configured to determine a body position of the user based on phase differences in the microphone and pressure sensor signals.

According to an embodiment, the one or more processors are further configured to determin, based on the vibration signals, one or more of a heart rate of the user, a respiration rate of the user, and movement of the user and to determine a sleep state of the user based on one or more of the heart rate of the user, the respiration rate of the user and movement of the user.

According to an embodiment, the one or more processors are further configured to issue an alarm in response to a determination that a predefined sleep state or predefined change in sleep state has been reached during an alarm period between a predefined start alarm time and a predefined end alarm time.

According to a further aspect there is provided a kit of parts for use in monitoring one or more physiological parameters of a user. The kit of parts comprises: at least one sensing unit comprising vibration sensors; a controller and at least one elastic tube configured to be embedded within a cushioning layer for supporting at least a portion of the user, wherein one end of the at least one elastic tube is sealed and the other end of the at least one elastic tube open for receiving the at least one sensing unit. The kit of parts is configured such that when the at least one sensing unit is received within the other end of the at least one elastic tube, a volume is defined by one or more inner walls of the tube and a surface of the at least one sensing unit for containing fluid such that the at least one elastic tube is configured to transmit vibrations from one or more sections of the at least one elastic tube to the at least one sensing unit for detection by the at least one vibration sensor. The vibration sensors include a microphone configured to generate a microphone signal based on detected vibrations and a pressure sensor configured to generate a pressure sensor signal based on the detected vibrations. The controller is configured to determine a body position of the user based on phase differences in the microphone and pressure sensor signals.

Embodiments as described herein may be implemented as devices, systems, kits of parts, methods or non-transitory computer readable media.

### BRIEF DESCRIPTION OF THE DRAWINGS

Arrangements of the present invention will be understood and appreciated more fully from the following detailed description, made by way of example only and taken in conjunction with drawings in which:
FIG. 1 shows a cross sectional view and a zoomed in cross sectional view of a sleep monitoring apparatus according to an embodiment;
FIG. 2 shows a cross sectional view of the sleep monitoring apparatus embedded within a cushioning layer;
FIG. 3 shows a sleep monitoring apparatus comprising a network of tubes according to an embodiment of the invention;
FIG. 4 shows a sleep monitoring apparatus according to an embodiment embedded within a cushioning layer forming part of a bed;
FIG. 5 shows a side view of the sleep monitoring apparatus of FIG. 4;
FIG. 6 shows an alternative embodiment in which tubes extend longitudinally down the length of a bed;
FIG. 7 shows a perspective view of the embodiment of FIG. 6;
FIG. 8 shows a typical graphical representation of a pressure signal detected by a sensing unit according to an embodiment;
FIG. 9 shows an intensity against time graph of the signal retrieved by the sensing unit in relation to heartbeat and breathing;
FIG. 10 shows a cross-sectional view of a sleep monitoring apparatus according to an embodiment of the invention;
FIGs. 11A and 11B show how the sleep monitoring apparatus may be arranged inside a mattress according to an embodiment of the invention;
FIG. 12 shows a side view of the sleep monitoring system of FIG. 11A;
FIGs. 13A - 13F show examples of different cross sections for the tube according to various embodiments;
FIG. 14 shows the sensing unit according to an embodiment;
FIG. 15 shows a manufacturing process for the integration of the sleep monitoring apparatus into a cushioning layer.
FIG. 16 shows a table detailing a method for determining a sleep level of the user according to an embodiment; and
FIG. 17 shows a flow chart for a method of determining when to activate an alarm based on determined sleep levels according to an embodiment;

### DETAILED DESCRIPTION

Embodiments described herein relate to systems for monitoring physiological parameters of users in bed and, in particular, of monitoring sleep.

In order to avoid requiring a user to wear, or be connected to, any monitoring system(s), the present embodiments integrate sensors into the bed itself. This provides a less intrusive means of monitoring users' sleep and avoids causing discomfort to the user through the application of wearable technology. In addition, as the sensors are integrated into the bed, the sensors can be provided with a more reliable power supply, either by sourcing power from the mains electricity supply (grid power), or by providing a larger battery than would otherwise be feasible on a wearable device due to size and comfort constraints. This reduces the risk of measurements being missed due to loss of power, which can be problematic due to the relatively long period of sensing and the fact that the users will usually be unaware that power has been lost, as they are asleep.

In general, there have been proposed two different approaches for monitoring sleep of a person on a bed. One of these is based on measuring sleep rhythms through sensors that are separated from the bed and the person being monitored, such as acoustic noise sensors or optical cameras. The other option is to place motion sensitive elements directly into contact with the body, or very close to body of the person being monitored. For the latter, sensors are placed, for example, under the sleeping lever of the mattress.

The drawback of the first solution - monitoring of sleeping person over distance - is its inexactness. It is difficult to obtain accurate measurements when the sensors are not located close to the person being sensed. The second method - placing sensors close to the body - enables more exact measurements, for example, the monitoring of even heart rhythms and breathing of a sleeping person. Having said this, placing sensors in close proximity to the body of the person being measured (e.g. directly under the sleeping surface) is problematic because they can easily break under mechanical stress caused by the persons on the bed. This is especially critical when long-term guaranties for the system may be requested. Adapting sensors to withstand the additional level of stress can greatly increase the complexity and cost of the sensors. Furthermore, integrating such sensors directly into the mattress is likely to negatively affect the comfort of the mattress.

Data on sleep can not only help to identify medical conditions, but it can also be used to control other user devices that might disturb the user during sleep. For example, some devices disturb sleep by causing noise or light when the user is asleep, or in the run-up to the user's bedtime. By obtaining high quality sleep data, smart devices can be controlled to improve the environment of the bedroom to optimise it in order to improve the user's sleep. For example, devices that activate motors or actuators would usually cause noise during this process. Therefore, the user's sleep can be improved if such devices are controlled so that they are switched on only when no one is sleeping in bed or only when user(s) are sleeping deeply (and therefore unlikely to be woken).

One of the example of problems where such smart switch-on-and-off devices are needed is related to poor sleep quality caused by dustiness of the air in the bedroom. People who have dust allergies are familiar with sneezing and other uncomfortable symptoms. Dust allergies also cause congestion (leading to breathing difficulties), or cause their eyes to itch or become red and watery. Approximately 10% of the population are allergic to air dusts, such as: pollens, molds, pet hairs, dust mites, etc. These symptoms effectively lower sleep quality. Therefore, air filtering that enables removal of dust particles, which are causing these problems, would enable to increase sleep quality largely. Unfortunately, air filtering for dust removal causes mechanical noise, which affect sleep quality negatively.

Due to the above-mentioned problems, there is a need for improved means of monitoring physiological parameters and conditions, and in particular sleep, in a more accurate and less intrusive manner.

Whilst the above issues are discussed with regard to monitoring sleep on a mattress, similar issues can be encountered when attempting to monitor physiological condition (e.g. heart rate, breathing rate, stress, etc.) via integration of sensing technology into other types of furniture, such as seating (armchair, sofa, etc). For instance, sensors may be integrated into the cushion or back support of a chair or other form of seat, to monitor physiological variables, such as breathing and heartrate, and physiological condition, such as stress. Having said this, direct integration of sensors close to the supporting surface of the seat can cause discomfort and increase mechanical stress upon the sensors.

The embodiments described herein aim to provide a solution for the problems mentioned above. Instead of placing sensors directly into the mattress (under the sleeping surface or into the seat under the seating surface), the present application proposes to lead signals (e.g. mechanical vibrations) out from the bed or seat, with the help of gaseous fluidic vibration conducting medium based collectors (conductors of mechanical vibrations) which we call signal collectors and which are placed under the sleeping/seating surface.

A signal collector is an elastic vessel filled with a vibration conducting fluidic, and preferably gaseous, medium, such as air. It can be made from a polymeric material, for example, rubber or PDMS. It can be a pipe that encloses a fluid (such as a gas or liquid) inside as a vibration conducting medium.

The system can make use of a gas or a liquid as vibration conducting mediums. A gas-based medium has advantages over a liquid-based medium, as liquid-based systems would be technically more problematic due to their lower durability, and the risk of the liquid evaporating over time if the signal collector is not effectively sealed. Signals, caused by outer force (e.g. a person on the bed) on the surface of collector are conducted by the fluid filling the reservoir (hollow region of the collector) and lead out from the mattress along the elongated collector, where they are detected by, for example, by pressure sensors. The signal collectors are formed of elastic material as this helps to transfer external mechanical vibrations into the conducting fluidic medium.

The signal collector can be used to lead mechanical vibrations, caused by a person sleeping on the bed, out from the mattress. When the signals are conducted out from the mattress, it is simple to connect sensors to the signal collector to measure the mechanical vibrations or pressure changes. This allows measurements to be taken outside of the mattress or at least at a distance from the human body so as to avoid mechanical stress on the sensors and to avoid negatively affective the comfort of the mattress. This configuration has many advantages compared to placing sensors directly under the sleeping surface:
- Locating the sensors outside of the mattress, or at the periphery of the mattress, makes it easier to connect the signal collector to the sensors
- The mechanical stress on the sensors is reduced, thereby reducing the risk of damage and allowing for the use of less durable (and therefore less expensive) sensors
- The solution is cost effective a single sensor can sense signals over a large sensing area via the use of the collector system spread under the sleeping surface.

Measuring of signals with the help of the collector placed between the signal source and sensor might result in some losses of signal quality and quantity. However, recent enhancements in sensor quality mean that sensors are often far more sensitive than needed for the purpose of sleep monitoring, including for example: acceleration sensors, pressure sensors, optical sensors, (optical) position sensors, etc. Still, signal collectors could also force small vibrations caused by a stronger force, by transferring the force into more intense mechanical motions before measurement.

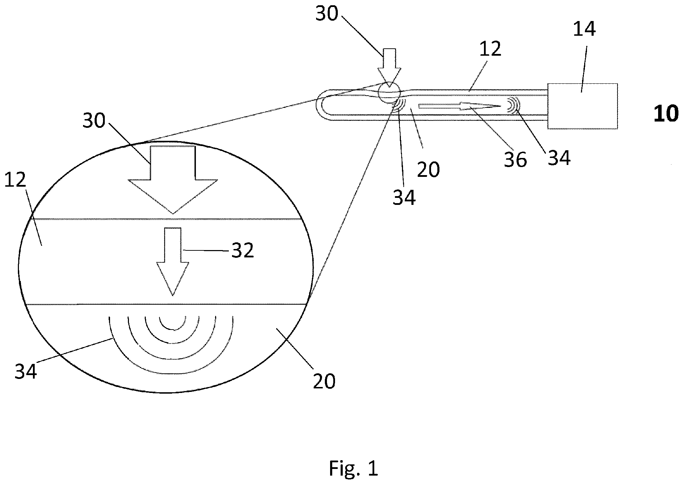

FIG. 1 shows a cross sectional view and a zoomed in cross sectional view of a sleep monitoring apparatus 10 according to an embodiment. The sleep monitoring apparatus 10 comprises a tube 12 and a sensing unit 14 containing fluid 20.

The tube 12 forms a signal collector for transmitting mechanical vibrations to the sensing unit 14. The tube 12 is elongated along a longitudinal axis. The tube 12 is closed on one end (the distal end) by the tube itself to hold the fluid 20, whilst the other end (the proximal end) opens onto the sensing unit 14 via a coupling section of the tube 12. The coupling section 12 is configured to couple with the sensing unit 14 so that the sensing unit 14 closes the open, proximal end of the tube 12 thereby containing the fluid 20 within an internal volume of the tube 12 (defined by inner walls of the tube 12). The sensing unit 14 may be coupled to the proximal end of the tube 12 via a sealing portion, locking mechanism or any other suitable means to prevent the fluid 20 from escaping the tube 12.

The fluid 20 may be a gas or liquid. Preferably, the fluid 20 is air.

The tube 12 is formed of a flexible material to enable the effective transfer of external pressure to the fluid 20. Preferably, the tube 12 is elastic. The tube may be made from materials such as rubber or polydimethylsiloxane (PDMS) or any other material that would be suitable to transmit the external force 30 as useful signals to the sensing unit 14. It is important that the tube 12 retains its initial size and shape as soon the external force is eliminated. Forming the tube 12 from elastic material provides resilience such that the tube 12 returns to its original shape upon removal of the force.

The sensing unit 14 comprises a pressure sensor, which detects pressure changes in the fluid 20. When an external force 30 is applied to the tube 12, the external force is transmitted to the fluid 20 through deformation of the wall of the tube 12. The external force 30 creates a vibration 34 inside the fluid, specifically a pressure change in the fluid 20. This vibration 34 is propagated along the length of the tube 12 until it reaches the proximal end of the tube 12 at which the sensing unit 14 is located. The sensing unit 14 then senses the vibration 34.

In one embodiment, the sensing unit 14 comprises a controller configured to determine physiological parameters from the sensed vibrations. In another embodiment, the sensing unit 14 comprises an output module (such as a wireless transmitter) for sending vibration measurements to an external system that is configured to determine physiological parameters from the sensed vibrations.

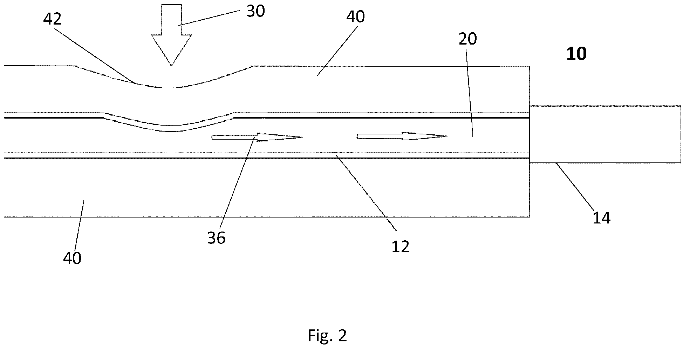

FIG. 2 shows a cross sectional view of the sleep monitoring apparatus 10 embedded within a cushioning layer 40. The sleep monitoring apparatus 10 is situated inside the cushioning layer 40 and underneath a contact surface 42. The cushioning layer 40 is a deformable and resilient layer for supporting a portion of a human body. The cushioning layer may be filled within cushioning material, may be filled with a cushioning fluid (such as air) and/or may comprise one or more elastic sheets or threads for providing support. The cushioning layer 40 may form of be part of, for instance, a mattress, or mattress topper within a bed or a cushion or back support of a chair. The contact surface 42 is a surface configured to receive pressure from a portion of the human body, in use.

When an external force 30 is applied to the contact surface 42 of the cushioning layer 40, the external force 30 is transmitted to the walls of the tube 12. Consequently, pressure change is induced in the fluid 20 due to the vibrations 34 of the tube 12 caused by the external force 30.

In the present embodiment, the sensing unit 14 is situated outside of the cushioning layer 40; however, as shall be described below, it may also be embedded within the cushioning layer 40. Ideally, the sensing unit 14 away from a supporting section of the cushioning layer 40 that is configured to receive the weight of the user. For instance, the sensing unit 14 may be located at a periphery of the cushioning layer 40. Nevertheless, the tube 12 is located under the supporting section to transfer vibrations to the sensing unit 14. In one embodiment, the sensing unit 14 is be placed at least 5 cm or more away from the supporting section.

The cushioning layer 40 can be (or form part of), but is not limited to: a mattress, a seat, a cushion or any other object configured to support or be urged against a significant or substantial part of a person's body for a prolonged duration of time. Ideally, the cushioning layer will be configured to support or be urged against a torso of the user, to aid in the detection of breathing rate and heart rate.

The contact surface 42 (or supporting surface) is any part of the surface of cushioning layer 40 that can be, but is not limited to: a sleeping surface, a seating surface, a lying surface, a resting surface or any other surface that serves to be in contact with a significant or substantial part of a person's body for a prolonged duration of time.

From the signals received by the sensing unit 14 from the vibrations 34 of the tube 12 due to the external force 30, the sleep monitoring apparatus 10 may determine physiological properties of the person in relation to sleep, such as body movements, body position (such as determination as to whether the person is lying on back, side or stomach), breathing rate and heart rate. Alternatively, measurements (or derived physiological parameters) may be exported (e.g. via a wireless connection) to an external system configured to determine these physiological properties.

Whilst the embodiment of FIG. 1 relates to a collector comprising a single longitudinal tube 12, the collector might be formed of a variety of shapes.

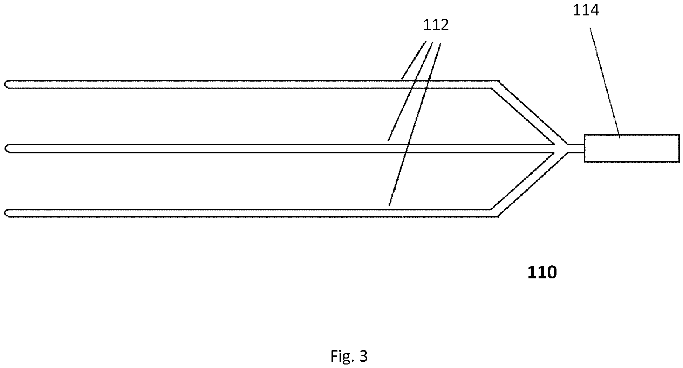

FIG. 3 shows a sleep monitoring apparatus 110 comprising a network of tubes 112 according to an embodiment of the invention. Three separate tubes 112 form a collector that is able to pick up and transfer vibrations from a larger area. The three tubes 112 are connected in parallel to a common node that is connected to the sensing unit 114 such that the network of tubes 112 are connected to each other to form a single volume. That is, the three tubes 112 are in fluid communication with each other and with the sensing unit 114. The tubes 112 are of the same dimensions in FIG. 3. Whilst three tubes are shown, it will be clear than any number of tubes may be implemented in any arrangement suitable for picking up and transmitting vibrations back to the sensing unit 114 (e.g. having multiple branches, branching at different sections, etc.)

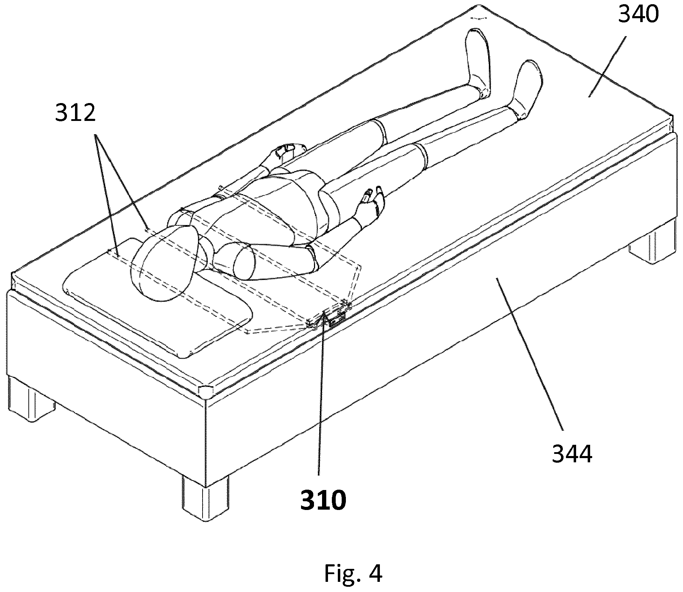

FIG. 4 shows a sleep monitoring apparatus 310 according to an embodiment embedded within a cushioning layer 340 forming part of a bed 344. The cushioning layer 340 (e.g. mattress) is placed on top of the bed 344 for a person to rest on. The cushioning layer 340 is configured to support the person at a supporting section located towards the centre of the upper contacting surface of the cushioning layer 340.

The sleep monitoring apparatus 310 is similar to that of FIG. 3; however, it comprises four tubes 312, rather than three. The tubes 310 are embedded within the cushioning layer 340, below the contact surface. The tubes 312 extend transversely across a substantial portion of the width of the cushioning layer 340 to provide a sensing area across the cushioning layer 340 over which vibrations may be detected. The tubes 310 are located towards a top end of the cushioning layer 340, so that they are located below a torso of the person once the person lies down on the bed 344 with their head towards the top end. This helps to enable the detection of heart rate and breathing rate.

Once the person comes into contact with (e.g. lies on) the sensing area, an external force 30 will be applied to the tubes 312 causing vibrations to be transmitted to the sensing unit 314.

Whilst the arrangement of FIG. 4 shows the sleep monitoring apparatus embedded within the bed in a specific arrangement, the sleep monitoring apparatus 310 may be placed anywhere within the cushioning layer to gather signals from the specific part of the bed. For instance, one or more tubes 312 may be integrated below an area for supporting the legs of a user to detect leg movement during sleep.

The vibration detected by the system may be mechanical and the sensing unit 314 may receive mechanical vibrations from the bed 344.

In one embodiment, the sensing unit further comprises a microphone. In some embodiments, the sensing unit uses both the pressure sensor and the microphone simultaneously. The sensing unit in some embodiments may use only one of a pressure sensor or microphone at any given time. In some embodiments, different signals may be captured by different sensors independently and/or simultaneously. For example, breathing and body movement can be detected by the pressure sensor(s) while heartbeat can be detected by the microphone.

The sensing unit may further comprise of other sensors that are capable of detecting vibrations, oscillations or impulses within the elastic signal collector. This also includes accelerometer(s). An example pressure sensor that can be used is a 40kpa MPS20N0040D pressure sensor.

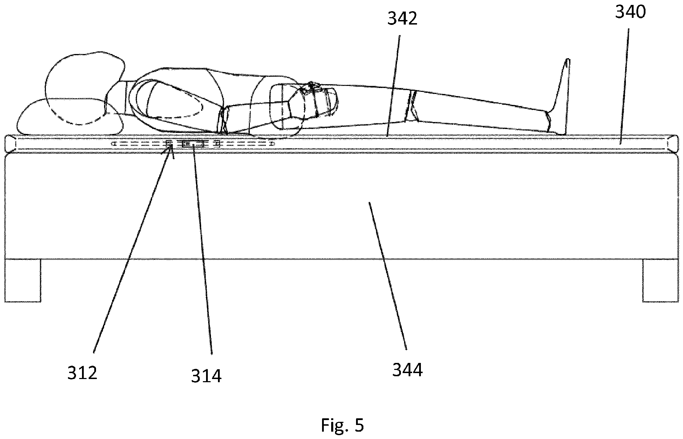

FIG. 5 shows a side view of the sleep monitoring apparatus 310 of FIG. 4. The sleep monitoring apparatus 310 may be configured to monitor the heart rate and/or breathing rate of a person lying down on the bed 344, as shown in FIG. 5. In the present embodiment, the sleep monitoring apparatus 310 is positioned within the cushioning layer 340, below in a region of the cushioning layer 340 configured to be located beneath the person's torso when the person lies on top of the cushioning layer 340.

This ensures that vibrations from the heart can be transferred to the sensing unit 314 for detecting heart rate. Equally, vibrations from breathing movements (e.g. movement of the diaphragm and rib cage) are transmitted to the sensing unit for detecting breathing rate. Both heart rate and breathing rate are good indicators of sleep level (e.g. deep sleep, light sleep, REM sleep or awake).

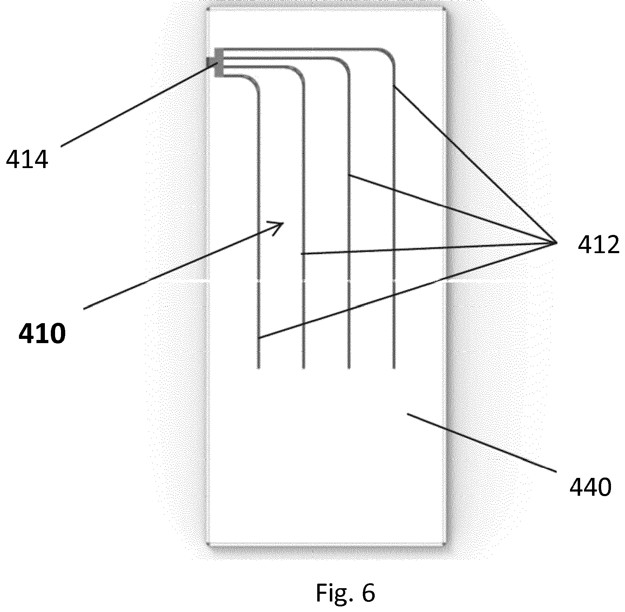

FIG. 6 shows an alternative embodiment in which tubes 412 extend longitudinally down the length of a bed. Cushioning layer 440 is in the form of a rectangular mattress. The tubes 412 comprise a bend along their length such that a larger proportion of the tube 412 may extend along the length of the cushioning layer 440, rather than the along width of the cushioning layer 440. The sensing device 414 is located towards one end of the cushioning layer 414, with the tubes 412 extending towards (and beyond) the centre of the cushioning layer 440.

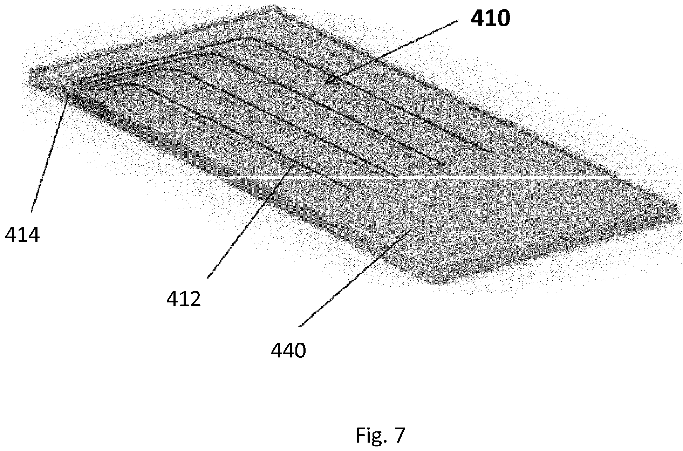

FIG. 7 shows a perspective view of the embodiment of FIG. 6. As shown here, the sensing unit 414 is embedded within a side of the cushioning layer 440.

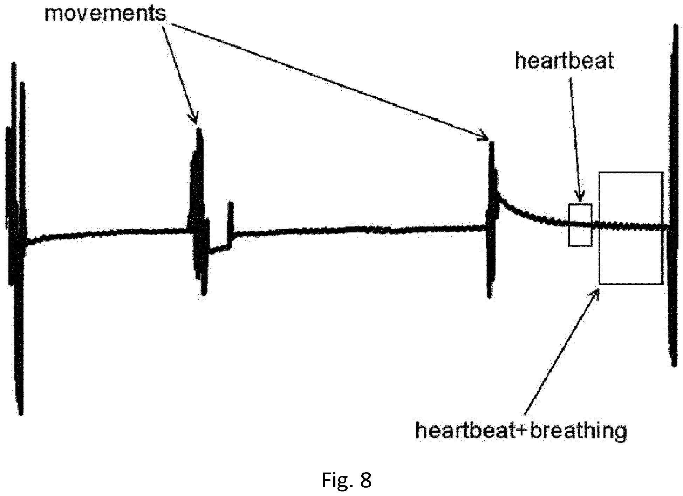

FIG. 8 shows a typical graphical representation of a pressure signal detected by a sensing unit according to an embodiment. This shows the characteristic signal patterns for body movement, heartbeat and breathing.

The length of the tube 12 influences the absolute signal amplitude received by the sensing unit 14. Different physiological properties of the person are captured and distinguished through the relative amplitudes of their signals.

FIG. 8 shows that body movements, such as turning of the body or movement of the head, legs, hands or shoulders have large amplitudes caused by their vibrations 34. The two largest peaks in beginning and end of the graphical representation relate to the person going to bed (lying down) and getting out of bed (getting up).

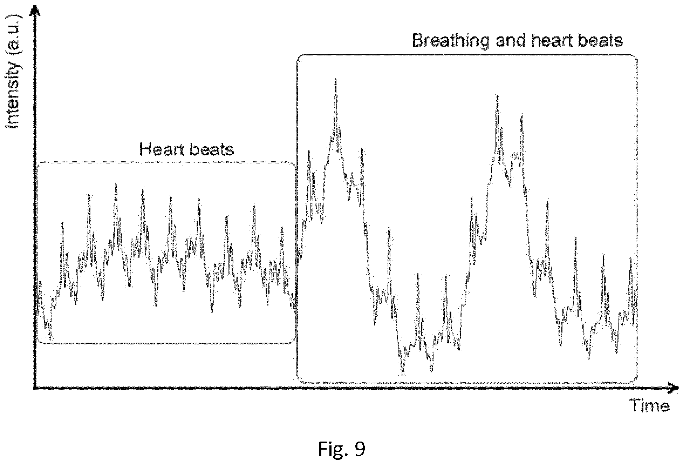

Breathing causes average amplitudes of vibrations. Heartbeat amplitudes are relatively small, but are still clearly detectable, as shown in FIG. 9.

FIG. 9 shows an intensity against time graph of the signal retrieved by the sensing unit 14 in relation to heartbeat and breathing.

Breathing is characterised by longer wavelength and higher amplitude oscillations compared to heartbeats. Heartbeat amplitude is approximately five times weaker than signals corresponding to breathing. FIG. 9 shows the intensity of the two signals being measured together. Heart rate is roughly six times higher than breathing rate.

In light of the above, the system is able to detect heartbeats, breathing cycles and body movements based on the amplitude and frequency of the detected vibrations. If vibration signal amplitude exceeds a first predefined limit then movement is detected. If vibration signal amplitude is less than the first predefined limit but above a second predefined limit (that is lower than the first), and if it falls within a first predefined range of wavelength or frequency, then breathing is detected. If the vibration signal amplitude falls below the second predefined threshold and falls within a second predefined range of wavelength of frequency, then a heartbeat is detected. The second predefined threshold range of wavelength is smaller than the first predefined range of wavelength (covers wavelengths that are lower). Conversely, if frequency is being monitored, then the second predefined threshold range of frequency is higher than the first predefined range of frequency (covers frequencies that are higher).

Based on detected movement, breathing and heartbeats, the system is configured to determine breathing rate, heart rate and rate of movement. This allows the system to characterise the level of sleep (or level of awakeness) of the user.

The combined operation of an embodiment shall now be described.

An embodiment includes a sleep monitoring system 10 comprising a cushioning layer 40 (such as a mattress or a seat), the sleep monitoring system 10 placed under a contact surface 42 and filled by fluid 20 (air in usual cases), used to collect and conduct (the tube 12) mechanical vibrations 34 inside the cushioning layer 40 to the remote (at least 5 cm or more away from signals source) sensing unit 14 for the purpose of detection of vibrations 34 caused by sleeping persons (see FIG.5).

When external forces 30 are influencing an outer side of the sleep monitoring system 10 wall they are transmitted through the wall and transformed into mechanical vibrations 34 of fluid, packed inside the tube 12 (see FIG.1). The sleep monitoring system 10, assembled from an elastic tube 12, is chosen in such a shape so that it transfers (or leads) vibrations from their original location to desired place(s) for the purpose of their detection by a sensing unit 14 (see FIG.1 and FIG. 2). The elastic tube 12 is integrated into the cushioning layer 40 where it is useful as a sleep monitoring system 10 to collect vibrations 34 or oscillations caused by body movements, including breathing and heartbeat, and transport these vibrations 34 along the elastic tube 12 away for the purpose of detection.

The sleep monitoring system 10 is assembled from one or more elastic tubes 12 made of rubber or polydimethylsiloxane (PDMS) or any other elastic material that is able to transmit the signals and transform them into mechanical vibrations 34 of fluid inside the tube 12. It is important that tubes 12 restore their initial size and shape as soon the external force 30 is eliminated, to ensure continued detection. The shape of the elastic tube 12 of the sleep monitoring system 10 can be any, but is usually elongated, to enable transmission of signals over distance into a sensing unit 14, as fluid that is packed inside the elastic tube 12 (air or any other stable gas or mixture of gasses) can be applied.

Such systems (combination of an elastic tube 12 and the fluid inside) can be used as a signal collector of mechanical vibrations to lead them over the distances in the form of vibrations 34 of fluid, away from the source of the vibrations 34 (e.g. a sleeping person on the cushioning layer 40). Thus, the sleep monitoring system 10 is used to collect signals and lead them out from the cushioning layer 40, for the purpose of detection. Detection can be implemented on the side of mattress or at least away from the body (e.g. more than 5 cm away from the body). This avoids damage to the sensing unit 14 by mechanical stress caused by the weight of the body, and avoids negatively affecting the comfort of the cushioning layer 40.

As the sleep monitoring system 10 is connected to vibration sensitive elements, when placed inside the cushioning layer 40, it can be uniquely used for detection and subsequently distinguish signals associated with body movements, body position (lying on back, side and stomach), breathing and/or heartbeat. An advantage of the sleep monitoring system 10 is that it allows the fragile electronics to be kept away from persons on the cushioning layer 40 for safety and durability as the persons can cause mechanical forces that can cause strain and even break fragile electronics. An additional advantage is that electronics are located away from the user, thereby eliminating any possible negative effects for human health caused by electromagnetic fields, caused by electric measurement systems or just to lower psychosomatics effects - for example, the worry that there would potentially be some negative health effects when sleeping near to an electronic device.

The elastic tube 12 forming the sleep monitoring system 10, is for example, an elastic pipe, which is placed between softening materials of the mattress so that motions, heartbeats and breathing of person on the cushioning layer 40 will cause mechanical force on its surface. Signal detection at the other end of the tube 12 can be achieved, for example, by a pressure sensor, microphone or some other sensor that is suitable for detection of mechanical vibrations 34. For the purposes of signal detection, more than one sensor can be connected to sleep monitoring system 10. The simultaneous use of different sensors, for example the co-use of microphone and pressure sensor, for example, would be useful to collect signals with different frequencies at the same time. This is important as different kinds of external forces 30 cause signals with different frequencies inside the collector. Thus, breathing can be detected just by pressure sensors while hearth rhythms can be detected also by microphone.

In one embodiment, the pipe forming the elastic tube 12 would be hermetic, but in alternative embodiments, the elastic tube 12 is not hermetic, for instance, it may have holes or pores inside its walls. Through those pores or holes, atmosphere (air) inside the pipe is in physical connection with atmosphere (air) outside the tube 12. The connection of inner and outer atmospheres could be useful as it enables to keep a base signal during the measurements close to corresponding value of ambient air pressure. Therefore, for example, when a person goes to bed then additional pressure inside the elastic tube 12, caused by outer force, is lost in reasonable time and further measurements can be made close to ambient air pressures. The latter would be very useful as it enables the maintenance of the pressure within the chamber within the optimum sensing range of the sensing unit 14 (this avoids excessively high pressures). Otherwise, when a person goes to bed or when he/she leaves the bed, it would cause pressure to be driven out of the sensors optimum sensing range. Thus, leaking through the wall of the tube 12 enables system to keep the sensor unit 14 operating within their optimum pressure range.

The elastic tubes 12 are to be integrated inside the cushioning layer 40 to a specific depth to get out optimal signals as well as to preserve softness of the cushioning layer 40. The exact depth of the sleep monitoring system 10 depends on the mechanical properties of sleep monitoring system 10 as well as the material of the cushioning layer 40.

The signal collector, comprising tubes 12 or a network of tubes 12, should be located close to the signal source, taking into account that the cushioning layer 40 remains between the body and elastic tubes 12 of the sleep monitoring system 10. Having said this, in some cases, for example, when using low elasticity materials, the sleep monitoring system 10 can be most effective when being in direct contact with a body, with no cushioning layer 40 between the body and collector (for instance, the signal collector might be located over the cushioning layer).

The exact form of the sleep monitoring system 10 position can vary. In an embodiment, the tubes 12 are placed perpendicular to the body in parallel jointed by T-junctions on the side of cushioning layer 40, approximately 2-3 cm from the surface of cushioning layer 40, under a layer of elastic foam within the cushioning layer 40. When integrated into a cushioning layer 40 like this, pressing the cushioning layer 40 on the top generates mechanical vibrations 34 of fluid (preferably air) inside the tubes 12. In addition to the tubes 12, a pressure sensor is utilised to detect signals (FIG. 8 and FIG. 9).

With regard to the sensing unit 14, any sensor that can detect elastic media vibrations, oscillations or impulses such a pressure sensor, microphone, accelerometer or similar can be used. In one embodiment, a 40kpa MPS20N0040D pressure sensor is used. The length of the pipe strongly influences absolute signal amplitude. From this point of view, body (e.g. head, leg, hand, shoulder) movements cause relatively large vibration amplitudes. Breathing causes relatively average vibration amplitude. Heartbeat amplitude remains relatively low, but still clearly detectable. Corresponding signals can be seen from FIG. 8 and FIG. 9.

### Example Use of an Embodiment

A soft elastic tube (8 mm in diameter, 2 mm in wall thickness, 1 m in length), made of rubber or polydimethylsiloxane (PDMS) or some other elastic material, filled by air as gaseous fluid is integrated into the mattress under the sleeping surface. The tube is packed between soft elastic filler materials of a mattress so that approximately 2 cm thick layer of soft material will cover the tube or tubes. Air is used as the signal-conducting medium inside the tube. The tube is placed between softening materials of the mattress such that vibrations caused by a person on the mattress will force the pipe to generate mechanical vibrations that will be transferred outside the mattress by the fluid inside the pipe. A pressure sensor, used for detection of signals, is placed outside the mattress inside the far end of the pipe.

Since the particular modifications and dimensions will be apparent to a person skilled in the art, the invention is not considered limited to the said example chosen for purposes of disclosure.

### Further Embodiments

The following description below relates to further embodiments of the invention.

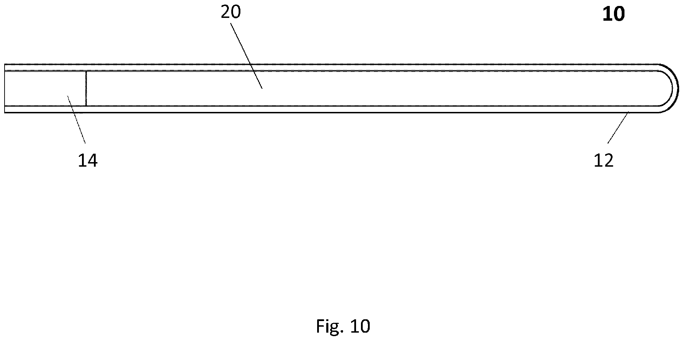

FIG. 10 shows a cross-sectional view of a sleep monitoring apparatus 10 according to an embodiment of the invention. Like numerals are used for corresponding parts relative to FIG. 1. The sleep monitoring apparatus comprises a tube 12 and a sensing unit 14. The tube 12 is fluid tight. One end of the tube 12 is sealed by the tube 12 itself whilst the other end is closed entirely by the insertion of the sensing unit 14. The tube 12 is sufficiently long to create a volume between the closed ends of the tube 12. The volume is filled with fluid 20. The fluid 20 may be any liquid or gas, but preferably air.

The tube 12 is sufficiently elastic enough such that when an external force 30 is applied to the outer surface of the elastic tube 12, it is able to return to its original shape after the external force 30 is eliminated. The Young's modulus for the elastic tube 12 may range from 0.1 - 100 MPa. The elastic tube 12 is preferably made out of a soft material in order for the mechanical vibrations to be captured effectively. Example materials of the tube 12 include, but are not limited to: natural rubber, synthetic rubber, silicone rubber, urethane and polydimethylsiloxane (PDMS).

The pressure sensor frequency capture range is 0 - 200Hz. This allows for the effective capture of breathing rate, heart rate. The microphone's frequency capture range is also 0 - 200Hz. The upper limit of 200Hz is the optimum range to detect vibrations from heartbeat, breathing and movement. Additionally, this filters out higher frequencies that can cause interference and avoids the detection of speech, which can lead to privacy issues. Speech at the specified range (0 - 200Hz) is largely incomprehensible as the speech information is generally transferred at higher frequencies.

In the present embodiment, the sensing unit 14 outputs the vibration measurements (from the microphone and from the pressure sensor) to an external device for processing. This may be via a wireless connection. The external device might be a computer, a mobile device such as a smartphone, or a central server. Nevertheless, in alternative embodiments, the processing is performed in a processor of the sensing unit 14.

The system is able to determine the user's sleep state, or level of alertness or stress, based on heart rate, breathing rate and movement rate over time. In addition to the changes in pressure, as discussed with regard to FIGs. 8 and 9, the detection of breathing rate and heartrate can be aided by detecting specific repetitive noise within breathing phase via the microphone (used to identify individual respiratory cycles or heartbeats). These noises may also be an indicator of certain diseases or sleep conditions.

Phase differences in microphone and pressure sensor signals are used to determine the body position of the user. The signals collected by the sensors depend on the relative position and orientation of the user on the cushioning layer 40. This allows detection of whether the user is on their back, on their stomach, on their left side or on their right side.

Depending on the particular position, the shape and amplitude of the resulting signal from heartbeats differs. Importantly, the signals from microphone and pressure sensor will be in phase or out of phase, depending on whether the person is lying on the back or stomach, respectively. These variations can be used to determine the position of the person lying on the mattress just from the signals collected by the tube 12, without any additional measurements.

For instance, the microphone signal can be utilised to identify individual heartbeats (or phases within a heartbeat/cardiac cycle). The pressure signal originating from heartbeat across the cardiac cycle shows the direction of movement of the heart (which is transferred into vibration in the bed). As the average location of the heart within a person is known, as well as the average motion of the heart during a cardiac cycle, the user's body position can be determined by comparing the phases of the sound and pressure signals. For example, the pressure signal measured when the person is lying on their right side has an opposite phase/direction to a signal measured when the person is on their left side. Accordingly, the sound signal can be used as a marker (trigger) in the analysis, with the reading from the pressure sensor showing the direction of movement of the right ventricle, i.e. the position of the heart in the bed.

Calibration of different positions can be carried out by the user to improve the detection of different lying positions.

Incorporation of multiple sensing units and/or multiple tubes in the mattress adds the possibility of also determining the position of person's limbs in the bed, for example, if the legs are stretched out or curled against the body. Multiple tubes enable vibration capture over a larger surface area of the mattress. These can either be connected to a single sensing unit (as discussed with reference to FIGs. 3-7, or each tube can be connected to a corresponding sensing unit. Providing a separate sensing unit for each tube allows easier separation of vibration signals from different sections of the mattress/cushioning layer.

Environmental noises can also be measured to improve the determination of the sleep state of the person. Environmental noises include, but are not limited to, noises from road traffic and electric appliances, such as air conditioner, fans and refrigerators.

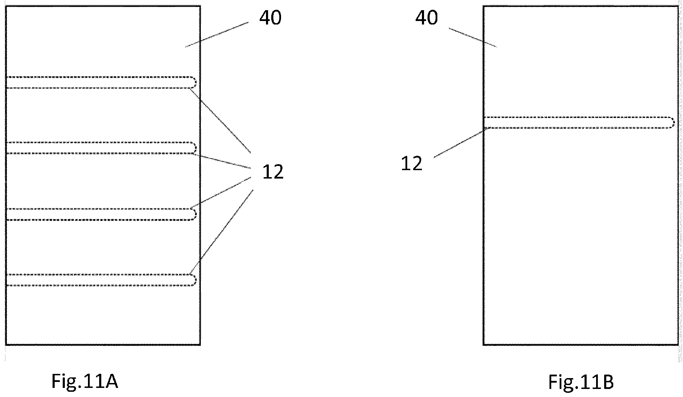

FIGs. 11A and 11B show how the sleep monitoring system may be arranged inside a mattress 40 according to an embodiment of the invention.

FIG.11A shows a system comprising multiple independent sleep monitoring apparatuses, whilst FIG. 11B shows a system comprising a single sleep monitoring apparatus.

Each sleep monitoring apparatus 10 is positioned inside the mattress and orientated to extend along the width of the mattress, with the sensing unit located at a side of the mattress. In the present embodiments, the sleep monitoring apparatuses comprise tubes 12 that extend along the full width of the mattress. Whilst four sleep monitoring apparatuses are shown in FIG. 11A, any number of apparatuses may be utilised to provide detection at desired locations within the mattress.

Larger mattresses/cushioning layers 40 may comprise multiple sensing units and/or sleep monitoring apparatuses to capture the mechanical vibrations more effectively. In addition, multiple sets of sensing units and/or sleep monitoring apparatuses may be utilised to capture vibrations from multiple users on the same cushioning layer 40, for instance, two columns of sensing units and/or sleep monitoring apparatuses might be utilised on a double bed to capture vibrations from two users. In this case, the sensing units for the two sets could be located on oppose sides of the cushioning layer, with the tubes extending towards the centre, on opposite halves of the cushioning layer 40.

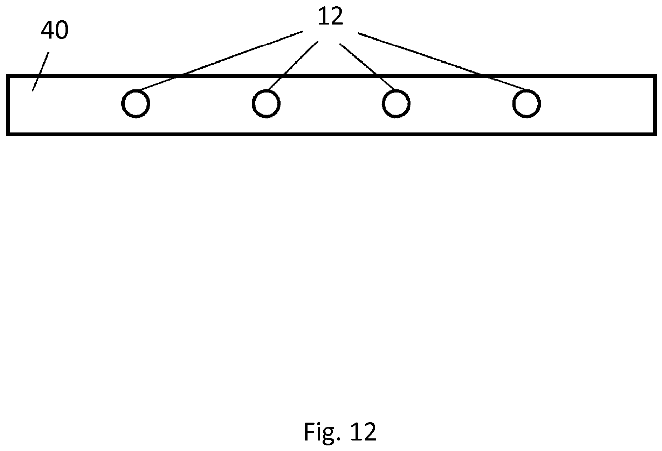

FIG. 12 shows a side view of the sleep monitoring system 10 of FIG. 11A. The tubes 12 arranged in regular intervals along the length of the cushioning layer 10. However, in some embodiments, the tubes 12 may be arranged such a way that the collection of vibrations 34 may be focused on certain contact areas 42. For example, more tubes 12 may be positioned at the top part of the mattress to collect vibrations 34 from the person's head and torso.

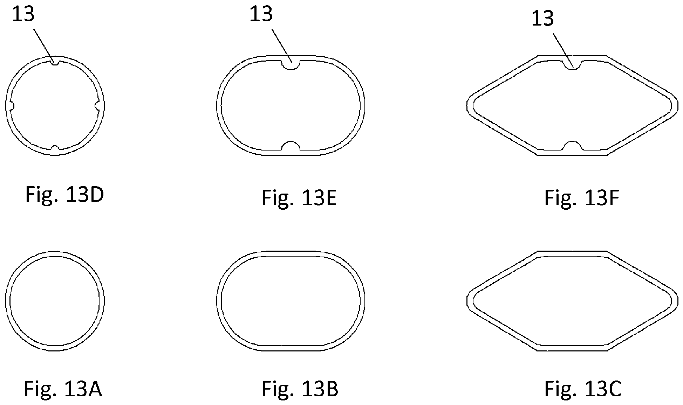

FIG. 13A - 13F show examples of different cross sections for the tube 12 according to various embodiments. The shape of the tube is important for transmitting vibrations 34 through pressure changes in the fluid 20. For example, a regular cross-section along the length of the tube helps to avoid reflections and/or noise when transmitting vibrations 34 through pressure changes in the fluid 20.

FIG. 13A shows a tube having a circular cross-section. FIG. 13B shows a tube having a cross-section having a central elongate section with two rounded ends. A similar embodiment might have an elliptical cross-section. FIG. 13C shows a tube having a cross-section having a central elongate section and two pointed ends.

FIGs. 13D-F show tubes having similar cross-sections to those of FIGs. 13A-C; however, inner protrusions are provided to reduce the risk of the tubes becoming stuck shut.

Due to the strength of the potential external forces 30 could be exerted on the tubes 12, the tubes 12 may be forced completely closed at certain points along the length of the tube. In this case, it is possible for the tube to stick shut when collapsed. This problem is solved by providing inner protrusions 13 inside the tube 12 to reduce the surface area of the inner wall that touches when the tube 12 is forced shut.

FIG. 13D shows a circular tube having four protrusions 13, spaced evenly around the circumference of the tube. FIG. 13E shows a tube similar to that of FIG. 13B but having two protrusions on opposite sides of the central elongate section. Equally, FIG. 13F shows a tube similar to that of FIG. 13C but having two protrusions on opposite sides of the central elongate section.

Each protrusion is a ridge that extends along the length of the tube. This provides a constant cross-section across the length of the tube, thereby reducing backward reflections within the tube.

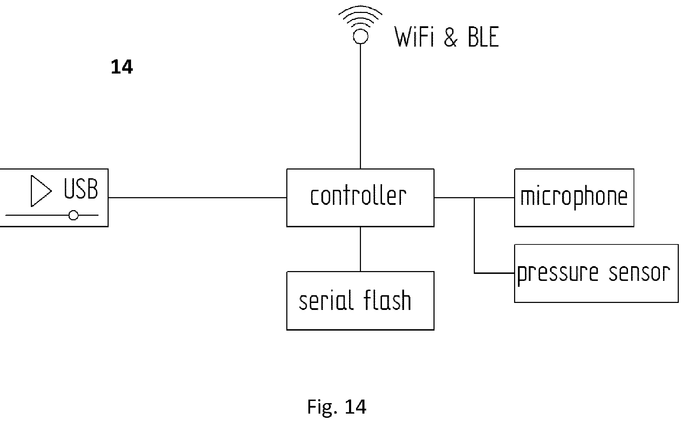

FIG. 14 shows a sensing unit according to an embodiment. The sensing unit 14 comprises a pressure sensor, a microphone, a controller, memory in the form of serial flash, a USB port and an input/output module in the form of Wi-Fi and Bluetooth modules.

The microphone is configured to detect vibrations in the form of sound within the tube. The pressure sensor is configured to detect pressure within the tube. The microphone and pressure sensor are connected to the controller and report their measurements to the controller. The controller is configured to store measurements in the memory (the serial flash) and to output measurements via the output module (e.g. the Wi-Fi or Bluetooth modules) or via the USB module. The system is powered via a battery or via a mains power supply, e.g. via the USB port.

Whilst the embodiment of FIG. 14 includes a serial flash, any form of memory might be utilised. Equally, whilst USB, Wi-Fi and Bluetooth modules, any appropriate form of output port output module might be utilised.

The memory stores computer executable instructions that, when executed, cause the controller to perform the functions described herein. Whilst the present embodiment outputs vibration measurements to an external system for analysis, alternative embodiments perform analysis of the measurements via the controller of the sensing unit.

The sensing unit 14 may a cross-section that corresponds to the internal cross-section of the tube 12 to allow the unit 14 to easily fit within the tube 12. Having said this, the sensing unit 14 is not required to have a cross-section that corresponds precisely to the internal cross-section of the tube 12 to work the invention, provided that at least a coupling section of the sensing unit 14 is configured to be inserted within the tube 12, for instance, by at least fitting within the tube 12 (having cross-section dimensions, e.g. width and/or height that are less than the inner diameter of the tube), if not having a corresponding cross-section to the tube 12. For instance, the coupling section might have a square or rectangular cross-section, with the tube 12 being sealed around the coupling section, e.g. via glue.

To assemble the system, the sensing unit 14 is first inserted into one end of the tube 12 that is closed on the other end. The sensing unit 14 is inserted deep enough to leave one end (in which the USB port is located) flush with the open end of the tube. The end of the tube with the inserted sensing unit 14 is thereafter hermetically sealed (e.g. by heat shrinking, using non-conducting glue, or both). The tube can be filled with various gasses before sealing. Alternatively, the sensing unit 14 may be held within the tube 14 via an interference fit; although this might result in air escaping the tube 14 during use.

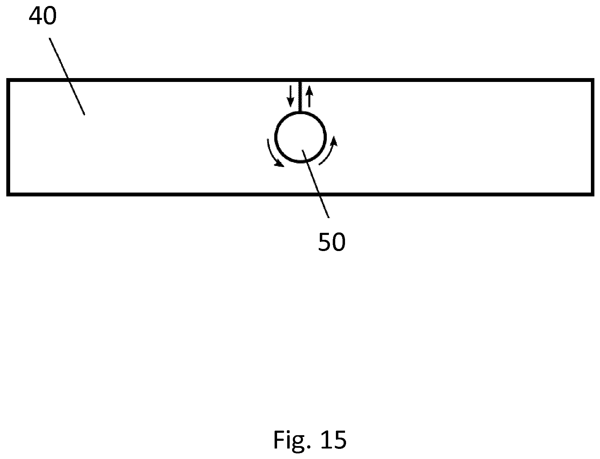

FIG. 15 shows a manufacturing process for the integration of the sleep monitoring apparatus 10 into a cushioning layer 40. The manufacturing process includes a cutting tool specifically designed for carving channels 50 into a cushioning layer 40.

Cushioning layers, such as mattresses, can be difficult to cut, due to their compressibility and resilience. Accordingly, a customized cutting tool is provided that makes the cutting of such cushioning layers easier.

The cutting tool comprises a diamond-coated wire of, for instance, steel. The wire is a formed into a loop and is driven via a motor (for instance, via a set of pulleys). By coating the wire with diamond (or with another suitably hard substance), a particularly hard and sharp edge can be provided for cutting.

The cutting tool functions in a similar manner to a band saw, with an exposed region having the wire running through it to provide an area for cutting the cushioning layer 40.

FIG. 15 shows a view down the length of a cushioning layer 40 (e.g. a mattress). In this view, the wire of the cutting tool runs perpendicular to the view (out of the page). A channel may be cut down the length of the cushioning layer 40 by urging the cushioning layer against the wire and moving it along the path shown in FIG. 15. The wire enters a top layer of the cushioning layer, forms a straight entry/exit path, then forms a loop/circle for cutting the channel, then exits through the entry/exit path.

In use, the cutting tool may be kept stationary (with the wire being driven by the motor) and the cushioning layer 40 may be urged against the wire to cut the desired shape (the shape of the channel 50 for receiving the tube). The cushioning layer 40 can be moved by hand or by a mechanical stage (e.g. computer controlled). Alternatively, the cushioning layer 40 may be kept stationary with the cutting tool being moved along a desired path to cut the channel 50.

The cushioning layer 40 can be compressed to enable cutting of mattress foams thicker than the length of the exposed cutting wire. In one embodiment, the channel 50 for the tube 12 is to be cut such that the distance between the top of the channel 50 and top of the cushioning element 40 is sufficient to avoid the tube 14 impacting the comfort of the cushioning layer, but shallow enough to ensure that vibrations are still picked up by the tube (e.g. at least 5 cm from the surface of the cushioning layer).

Finally, the tube 12 is inserted into the channel 50 in the cushioning element 40. The tube 12 can be freestanding (e.g. secured via an interference fit) in the channel 50 or alternatively, the channel 50 or tube 12 walls can be covered with glue prior to inserting the tube, in order to fix its position to avoid unwanted shifting and collapse of the tube 12 during the use of the cushioning layer 40.

The entry/exit point used for cutting the channel 50 can be left open (for instance, with the tube 12 being secured via glue within the channel 50 or an interference fit within the channel 50, or with the cushioning layer 40 being otherwise sealed, such as through containment within an outer cover). Alternatively, the entry/exit path can be sealed shut, e.g. via glue.

### Analysis of Raw Data and Data Streaming

The controller of the sensing unit is able to communicate directly with an external computing system, such as a mobile device, computer or server, through wireless communication, e.g. Bluetooth or Wi-Fi communication. In one embodiment, the sensing unit communicates with a user's mobile device (e.g. a smartphone). If the external device is not in range, or connection is lost through another means, then the data is stored and then transmitted to the external device when connection is restored.

The signal may be processed on the sensing unit 14, e.g. to remove noise and to compress the signal, and the processed signal can be sent to the external device for tasks that require additional computing power. In some alternative embodiments, a raw, unprocessed feed of data is sent to the external device for more precise analysis for the sensed data.

It is also possible to detect heart and breathing related diseases from the signals picked up by the sensing unit 14. The detection of these conditions may require more computing power than provided by the on-chip microprocessor in the sensing unit 14, and comparisons with external databases might be necessary. For example, spectral analysis may be needed, which requires a Fourier transform of the raw sensor signal.

Additionally, as these disease conditions manifest themselves as intricate and sometimes small deviations in sound spectra compared to normal healthy state, the raw data used for such analysis should have minimal noise.

In order to carry out such analysis, the signal from the sensing unit 14 is monitored by the software implemented on the controller overnight. To avoid exporting or storing large quantities of data, the system might monitor the raw data and only export or store a predetermined period (e.g. a short, burst) of raw data if certain conditions are met (e.g. when a good quality of signal is detected, and/or when a specific sleep level is detected).

The duration of the burst may be up to five minutes. Analysis of the raw data can take place in the external device or the data can be sent to a remote server to access additional computing power. Due to the high data rate of the raw data, it should be ensured that optimal conditions are present for collecting high quality data, so as not to waste energy and storage space for data that might not be relevant for further analysis.

Four parameters are monitored to determine the start of a data stream session and when all four conditions pass threshold values, a data stream is initiated. The thresholds may vary, depending on whether heart or breathing sounds are to be analysed.

The four parameters to be met for sending a data stream to a phone or remote server for heart/breathing condition analysis are:
(1) sleep state is deep sleep;
(2) there have been no movements in a predetermined period of time (e.g. the last five minutes);
(3) moving average root-mean-square (RMS) value of the signal has not changed by more than a predefined amount over a predetermined period of time (e.g. 20% in the last five minutes); and
(4) heart/breathing rate is detected.

The times and percentages in conditions (2) and (3) can vary (e.g. 1-20 minutes and 5-50%). Once these conditions are satisfied, the sensing unit 14 shall record and export the signal (e.g. over a predefined period or burst) for further analysis, for instance, exporting to memory and/or to an external device.

### Heart Rate Measurement

To detect heart rate, the frequency range of 10-30 Hz is filtered out from the raw heartbeat signal (e.g. via a band-pass filter). That is, frequencies outside of the range of 10-30 Hz are suppressed. The resulting heartbeat signal (over the 10-30 Hz range) is smoothed with a moving average method. Two passes of smoothing may be performed for improved performance. Local maxima are detected from the resulting signal. Every heartbeat gives two peaks (local maxima), one of them is primary and has a higher amplitude, and the other one has lower amplitude. The lower secondary peaks are removed and primary peaks are used to determine heart rate. That is, each primary peak represents a corresponding heartbeat. The time between primary peaks can be determined and utilised to determine frequency (e.g. by dividing the number of primary peaks with the time over which the primary peaks are measured). A moving window may be utilised to average out the heart rate over the window.

### Breathing Rate Measurement

Breathing rate measurement is performed without frequency range cutting. The raw signal is smoothed with the above-mentioned moving average method (e.g. using two passes). Peaks (local maxima) are identified for calculating breathing rate. For determining peaks, peaks that have an amplitude below a certain threshold amplitude and/or a frequency above a certain threshold frequency are disregarded as noise. This is achieved by determining the relative amplitudes and time separation between peaks so that peaks relating to small, high frequency fluctuations (i.e. that are unlikely to relate to breathing) are filtered out. The peaks within the filtered signal are then utilised to determine breathing rate. That is, each peak represents a corresponding breath (a single cycle of breathing). The time between peaks can be determined and utilised to determine frequency (e.g. by dividing the number of peaks with the time over which the peaks are measured). A moving window may be utilised to average out the breathing rate over the window.

### Sleep Level Classification

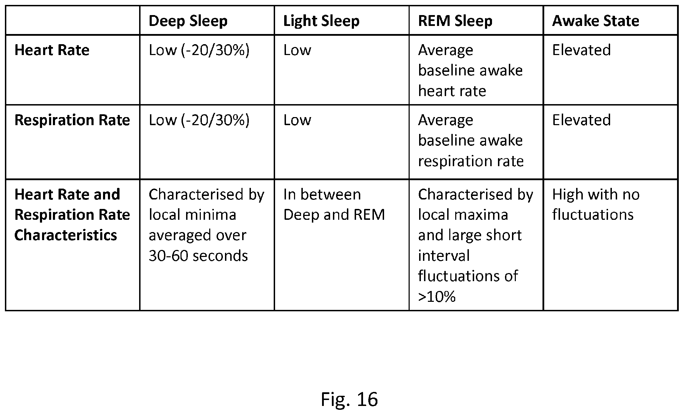

FIG. 16 shows a table detailing a method for determining a sleep level of the user according to an embodiment. A person goes through several stages of sleep throughout the night, broadly categorized as light sleep, deep sleep and rapid eye movement (REM) sleep. The sleep stages are cyclic and vary from light sleep to deep sleep and back to light sleep. At the end of each such cycle, REM sleep occurs. Each stage of sleep is associated with changes in heart rate and respiration rate. Periods of wakefulness can also occur. Compared to the awake state, light and deep sleep are characterized by lowered heart rate and respiration, with deep sleep having the lowest values (approximately 20-30% lower than awake).

The baseline awake heart and respiration rates for each individual user can be determined at the time the person goes to bed, before falling asleep. Long-term average awake heart and breathing rates can be recorded for using as a parameter for determining sleep states. During REM sleep, the heart and respiration rates increase to levels comparable to awake state, accompanied by above average variations in both rates. Following the decrease and increase of heart rate and respiration, and taking into account larger variation of both during REM sleep stage, sleep stages can be assigned. Deep sleep stages correspond to the local minima in the heart and respiration rate curves, averaged over 30-60 second intervals. REM sleep similarly corresponds to local maxima accompanied by large short interval fluctuations of >10%. Light sleep falls between deep and REM sleep in terms of heartrate and breathing rate. Being awake is characterized by elevated heart rate and respiration compared to light and deep sleep, but without fluctuations characteristic to REM sleep.

From this data and data obtained by the sensing unit 14, software on the external device, or on the sensing unit, is able to determine a curve of sleeps states (e.g. deep sleep, light sleep, REM sleep, awake).

In light of the above, a first set of thresholds may be set for the heart rate and/or breathing rate. If the heartrate and/or breathing rate are above this first threshold, and the variability in heartrate and/or breathing rate is below a corresponding threshold variability, then the system might classify the user as awake. If the heartrate and/or breathing rate are above the first threshold, and the variability in heartrate and/or breathing rate is above the corresponding threshold variability, then the system might classify the user as in REM sleep. If the heartrate and/or breathing rate are below the first threshold, but above a second threshold (that is less than the first threshold), then the system might classify the user as in light sleep. If the heartrate and/or breathing rate are below the second threshold then the system might classify the user as in deep sleep.

The system may classify the user as falling within one of the above classes if one or both of the heartrate and breathing rate fall within the corresponding limits described above.

### Method for Determining When to Wake Sleeping Person

The systems described here might be applied to determine the optimum time to wake a sleeping person (via an alarm) based on the sleep state of the person. Waking up during deep or REM sleep can cause a person to feel groggy and disoriented. Accordingly, there are advantages to monitoring sleep state to ensure that the user is woken at the appropriate time within the sleep cycle in order to ensure that they wake up feeling well rested.

A user can set a preferred time window for wake up time, specifically the earliest and latest time an alarm should sound, which can also be referred to as the start and end time, respectively. The predefined end time ensures that the user does not over sleep and miss any prearranged appointments. The predefined start time ensures that the amount of sleep is maximised whilst also allowing a period of time for selecting the most appropriate time to wake the person based on sleep state.

The sleeping person's stages of sleep are monitored throughout the night. The preferred time to wake up a person is once the person has returned to light sleep after completion of REM sleep. An incomplete and interrupted REM stage can result in a sensation of sleepiness for an extended period after wakeup.

Alternatively, if the above sleep state transition (REM to light sleep) is not observed, then it is still preferable to wake the user during light sleep than during deep or REM sleep, as this would still provide reduced drowsiness in the user.

Accordingly, if the user is in, or enters, REM sleep during the pre-defined wake up time interval, but no later than 15 minutes before the end time, the alarm will sound as soon as the user completes the REM stage and enters into light sleep stage or at the pre-defined latest alarm time, whichever comes first.

If the user is in light sleep stage within a predefined period (e.g. 15 minutes) before the pre-defined end time (has not yet been in REM sleep during the alarm interval), the alarm will sound 15 minutes before the latest time to avoid entering REM stage for a short period of time.

If the user is in deep sleep 15 minutes before the pre-set end time (has not yet been in REM sleep during the alarm interval), the alarm will sound as soon as the user enters light sleep stage or at the pre-set latest alarm time, whichever comes first.

The 15 minute time-point is indicative and can be varied for example in the range of 5-30 minutes. The difference between earliest and latest time that a user can set should be equal or greater than this time-point.

A snooze function can be added to the alarm, which when activated after the alarm sounds, will stop the alarm and sound it again after certain pre-set time (e.g. 1-15 minutes).

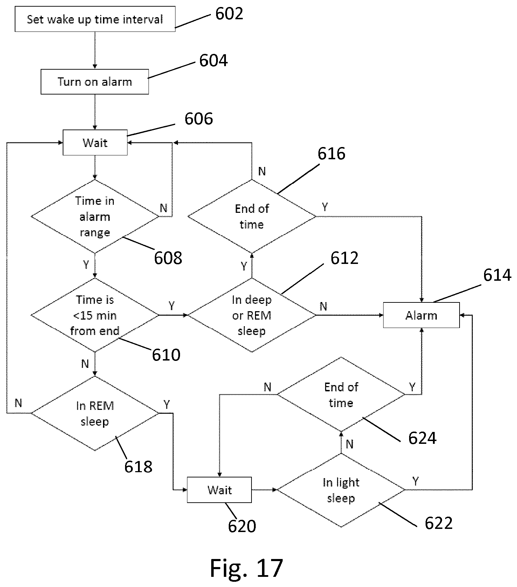

FIG. 17 shows a flow chart for a method of determining when to activate an alarm based on determined sleep levels according to an embodiment.

This method may be implemented within a controller within the sensing unit or may be implemented within an external device, such as a user's smartphone, that either receives vibration measurements and determines the sleep level itself, or receives reports of sleep level from another device (that bases these determinations of vibration measurements).

A first step 602 comprises the setting of a wake up time interval. This is the interval (an alarm period) over which the person wishes to be woken up by the sleep monitoring system 10. An end time is set up to define the latest time the alarm can be issued. A start time is set up to define the earliest time the alarm can be issued. The start time and end time are received via inputs from the user (e.g. via an input device such as a key board or touch screen, or via a connection with a further device, such as via wireless communication).

The system then receives an input to activate the alarm functionality 604. This instructs the system to monitor sleep activity and time, relative to the alarm period, and to issue an alarm to wake the user once the required criteria have been met.

The system then waits 606, monitoring the current time, until the start of the alarm period is reached (i.e. until the current time is equal to or later than the start time).

The system determines whether the current time is within the alarm period 608. If not then the system returns to step 606 to continue to wait. If the current time is within the alarm period 608 then the system then determines whether to issue an alarm to wake the user up.

The system then determines if the current time is less than a predetermined period (e.g. 15 minutes) from the end time 610. The predetermined period is shorter than the alarm period, so this step is only executed if the current time is in the alarm period. This step checks if the wake up time interval is nearing its end, in which case the system issues an alarm whenever the user is outside of deep or REM sleep, instead of waiting for a change from REM to light (as described below) to avoid the user entering REM sleep and not leaving REM sleep before the end time. 15 minutes is an arbitrary time period, although it is chosen as indicative of a time appropriate for avoid REM sleep from continuing on to the end time. Other lengths of predetermined periods may be used to achieve this function.

If the current time is within the predetermined period, then the system of determines if the person is in deep sleep or REM sleep 612. If not (e.g. the user is awake or in light sleep), then the system issues an alarm 614. If the user is in deep or REM sleep, then the system determines if the end time has been reached 616. If not, then the system loops back to step 606 and the method repeats. If the end time has been reached, then the alarm is issued 614.

If, during step 610, it is determined that the time is not within the predetermined period, then the system determines whether the user is in REM sleep 618. If not, then the system loops back to step 606. If the user is in REM sleep at this point, then the system waits 620, to see if the user will leave REM sleep before the end time.

The system checks whether the user has entered light sleep 622. If so, then the alarm is issued 614. If not, then the system determines whether the end time is reached 624. If so, then the alarm is issued 614. If not, then the system loops back to step 620 to continue to wait to see if the user will enter light sleep.

When an alarm is issued, it may either be an alert that is physically transmitted by the system (e.g. a sound and/or light) or it may be an electronic alert that is issued to another device to wake up the user (e.g. a wireless transmission to an alarm to issue a sound and/or light to wake up the user).

The ordering of the above steps are not necessarily the only order in which embodiments may be implemented. Some steps may be interchangeable and still achieve the same technical effect.

In light of the above, embodiments of the present invention provide a sleep monitoring system that is configured to detect mechanical vibrations for determining one or more physiological parameters (such as heart rate or respiration rate) and/or one or more physiological states (such as sleep level) of a person situated on a cushioning layer. This is achieved by providing a fluid-filled elastic tube connected to a sensing unit that can detect vibrations in the fluid caused by an external force on the elastic tube. A controller (either within the sensing unit or in an external device) is then able to determine the physiological state of the person depending on the user's heart rate, respiration rate, body position and/or movement based on the vibration sensed in the elastic tube. By providing a signal collector in the form of an elastic tube, vibrations are transmitted away from the body of the user, thereby allowing the sensing unit to be located away from the body, protecting the sensing unit from mechanical stress and avoiding any negative impact on the comfort of the cushioning layer that might be provided through the embedding of a hard sensing unit close to the user's body within the cushioning layer.

Further embodiments comprise an alarm system for waking up a sleeping person depending on the physiological state of the person, wherein the physiological state of the person can include stages of sleep. Stages of sleep include: deep sleep, light sleep, REM sleep and awake; which are also determined based on the vibrations of the fluid. By making use of the sleep state of the person, the alarm may be timed to wake the user at the most optimum point within the sleep cycle to avoid drowsiness.

It is noted that whilst most of the embodiments discussed above have been related to sleep monitoring, the above mentioned data and sleep monitoring apparatus 10 may be used for different types of physiological monitoring and utilised for different users, including, but not limited to:
- Sleep monitoring for personal use (health and lifestyle)
- Sleep monitoring for controlling external equipment - turning off equipment that might disturb sleep via sounds or light (e.g. fans, air conditioning, etc.) during light sleep stage
- Monitoring of elderly (personal and for caregivers)
- Monitoring of meditation (e.g. mindfulness)
- Monitoring of diseases and health problems (e.g. epilepsy, insomnia, snoring, sleep apnoea, heart attack, stroke)

Whilst the embodiments described herein have been described with reference to implementation within a bed or mattress, the methods described herein are applicable to other forms of cushioned furniture, or cushioned products. For instance, embodiments might be integrated within the backrest of a chair to monitor heart rate, breathing rate, movement, etc. This could be useful for determining stress levels and levels of awakeness. Equally, the embodiments might be implemented into the padding of a backpack to monitor heart rate and breathing rate. More generally, the embodiments can be implemented in any cushioning layer that is configured to be urged against the body of a user (or have a user's body urged against it), and in particular, against the torso of a user (for improved detection of respiration and heartrate).

While certain arrangements have been described, the arrangements have been presented by way of example only, and are not intended to limit the scope of protection. The concepts described herein may be implemented in a variety of other forms. In addition, various omissions, substitutions and changes to the specific implementations described herein may be made. The scope of protection is defined in the following claims.

## Claims

1. A system for use in monitoring one or more physiological parameters of a user, the system comprising:
at least one sensing unit (14) comprising vibration sensors including a microphone configured to generate a microphone signal based on detected vibrations and a pressure sensor configured to generate a pressure sensor signal based on the detected vibrations;
at least one elastic tube (12) configured to be embedded within a cushioning layer for supporting at least a portion of the user, wherein one end of the at least one elastic tube (12) is sealed and the other end of the at least one elastic tube (12) is closed by the at least one sensing unit (14) so as to form a volume that is filled with fluid and that is defined by one or more inner walls of the tube and a surface of the at least one sensing unit (14), wherein the at least one elastic tube (12) is configured to transmit vibrations from one or more sections of the at least one elastic tube (12) to the at least one sensing unit (14) for detection by the vibration sensors; and
a controller configured to determine a body position of the user based on phase differences in the microphone and pressure sensor signals.

2. The system of any preceding claim, wherein the at least one elastic tube (12) has a uniform cross-section along its length and/or wherein the at least one elastic tube (12) comprises one or more indents along the one or more inner walls of the tube to help prevent the tube sticking shut when crushed.

3. The system of any preceding claim, wherein the at least one elastic tube (12) comprises one or more indents along the one or more inner walls of the tube to help prevent the tube sticking shut when crushed, wherein the one or more indents are in the form of one or more ridges running along the length of the tube and comprise a plurality of evenly spaced indents around the circumference of the tube.

4. The system of claim 1 or claim 2, wherein:
the controller forms part of an external device and wherein the at least one sensing unit (14) is configured to send data relating to the detected vibrations to the external device for processing by the controller; or
the controller forms part of the sensing unit (14).

5. The system of any preceding claim, wherein the controller is further configured to determine, based on the detected vibrations, one or more of a heart rate of the user, a respiration rate of the user and movement of the user.

6. The system of claim 5, wherein the controller is configured to determine the heart rate of the user, wherein determining the heart rate comprises:
filtering the detected vibrations to form filtered vibration data over a predefined frequency range;
detecting local maxima in the filtered vibration data; and
determining the heart rate based on the detected local maxima.

7. The system of claim 5 or claim 6, wherein the controller is configured to determine the respiration rate of the user, wherein determining the respiration rate comprises:
detecting local maxima in the detected vibrations; and
determining the respiratory rate based on the detected local maxima.

8. The system of any of claims 5-7, wherein the controller is configured to determine movement of the user based on the detected vibrations, wherein determining movement of the user comprises determining whether an amplitude of the detected vibrations exceeds a predefined amplitude threshold.

9. The system of any of claims 5-8, wherein the controller is configured to determine a sleep state of the user based on one or more of the heart rate of the user, the respiration rate of the user and movement of the user.

10. The system of claim 9, wherein the controller is configured to perform one or more of the following:
determine that the user is awake in response to determining that one or both of the heart rate and respiration rate are above a corresponding first threshold and variation in one or both of the heart rate and respiration rate is below a corresponding variation threshold;
determine that the user is in rapid eye movement, hereinafter referred to as REM, sleep in response to determining that one or both of the heart rate and respiration rate are above the corresponding first threshold and the variation in one or both of the heart rate and respiration rate is above the corresponding variation threshold;
determine that the user is in light sleep in response to determining that one or both of the heart rate and respiration rate are below the corresponding first threshold and above a corresponding second threshold that is less than the corresponding first threshold; and
determine that the user is in deep sleep in response to determining that one or both of the heart rate and respiration rate are below the corresponding second threshold.

11. The system of claim 9 or claim 10, further comprising an alarm system that is configured to issue an alarm in response to a determination that a predefined sleep state or predefined change in sleep state has been reached during an alarm period between a predefined start alarm time and a predefined end alarm time.

12. The system of claim 11, wherein the alarm system is configured to perform one or more of the following:
issue the alarm in response to a determination that the user has transitioned from REM sleep to light sleep during the alarm period;
issue the alarm in response to a determination that a current time is within a predefined period from an end alarm time and the user is in light sleep during the alarm period, the predefined period being shorter than the alarm period; and
issue an alarm in response to the end alarm time being reached and the predefined sleep state or predefined change in sleep state has not been detected during the alarm period.

## Patentansprüche

1. System zur Verwendung in der Überwachung eines oder mehrerer physiologischer Parameter eines Benutzers, wobei das System umfasst:
mindestens eine Sensoreinheit (14), die Vibrationssensoren, welche ein Mikrofon beinhalten, das dazu konfiguriert ist, auf Basis von detektierten Vibrationen ein Mikrofonsignal zu erzeugen, und einen Drucksensor umfasst, der dazu konfiguriert ist, auf Basis der detektierten Vibrationen ein Drucksensorsignal zu erzeugen;
mindestens einen elastischen Schlauch (12), der dazu konfiguriert ist, innerhalb einer Polsterschicht zum Abstützen mindestens eines Teils des Benutzers eingebettet zu werden, wobei ein Ende des mindestens einen elastischen Schlauchs (12) versiegelt ist und das andere Ende des mindestens einen elastischen Schlauchs (12) durch die mindestens eine Sensoreinheit (14) verschlossen ist, um ein Volumen zu bilden, das mit Fluid gefüllt ist und das durch eine oder mehrere Innenwände des Schlauchs und eine Fläche der mindestens einen Sensoreinheit (14) definiert ist, wobei der mindestens eine elastische Schlauch (12) dazu konfiguriert ist, Vibrationen von einem oder mehreren Teilstücken des mindestens einen elastischen Schlauchs (12) zur Detektion durch die Vibrationssensoren an die mindestens einen Sensoreinheit (14) zu übertragen; und
eine Steuereinheit die dazu konfiguriert ist, eine Körperposition des Benutzers auf Basis von Phasenunterschieden in den Mikrofon- und Drucksensorsignalen zu bestimmen.

2. System nach einem vorstehenden Anspruch, wobei der mindestens eine elastische Schlauch (12) entlang seiner Länge einen einheitlichen Querschnitt aufweist und/oder wobei der mindestens eine elastische Schlauch (12) entlang der einen oder der mehreren Innenwände des Schlauchs eine oder mehrere Einkerbungen umfasst, die helfen, zu verhindern, dass der Schlauch zuklebt, wenn er zusammengedrückt wird.

3. System nach einem vorstehenden Anspruch, wobei der mindestens eine elastische Schlauch (12) entlang der einen oder der mehreren Innenwände des Schlauchs eine oder mehrere Einkerbungen umfasst, die helfen, zu verhindern, dass der Schlauch zuklebt, wenn er zusammengedrückt wird, wobei die eine oder die mehreren Einkerbungen in Form einer oder mehrerer Rippen vorliegen, die entlang der Länge des Schlauchs verlaufen, und eine Vielzahl von gleichmäßig beabstandeten Einkerbungen um den Umfang des Schlauchs herum umfassen.

4. System nach Anspruch 1 oder Anspruch 2, wobei:
die Steuereinheit Teil einer externen Vorrichtung bildet und wobei die mindestens eine Sensoreinheit (14) dazu konfiguriert ist, Daten, die sich auf die detektierten Vibrationen beziehen, zur Verarbeitung durch die Steuereinheit an die externe Vorrichtung zu senden; oder
die Steuereinheit Teil der Sensoreinheit (14) bildet.

5. System nach einem vorstehenden Anspruch, wobei die Steuereinheit weiter dazu konfiguriert ist, auf Basis der detektierten Vibrationen eines oder mehrere von einer Herzfrequenz des Benutzers, einer Atemfrequenz des Benutzers und Bewegung des Benutzers zu bestimmen.

6. System nach Anspruch 5, wobei die Steuereinheit dazu konfiguriert ist, die Herzfrequenz des Benutzers zu bestimmen, wobei das Bestimmen der Herzfrequenz umfasst:
Filtern der detektierten Vibrationen, um gefilterte Vibrationsdaten über einen vordefinierten Frequenzbereich zu bilden;
Detektieren von lokalen Maxima in den gefilterten Vibrationsdaten; und
Bestimmen der Herzfrequenz auf Basis der detektierten lokalen Maxima.

7. System nach Anspruch 5 oder Anspruch 6, wobei die Steuereinheit dazu konfiguriert ist, die Atemfrequenz des Benutzers zu bestimmen, wobei das Bestimmen der Atemfrequenz umfasst:
Detektieren von lokalen Maxima in den detektierten Vibrationen; und
Bestimmen der Atemfrequenz auf Basis der detektierten lokalen Maxima.

8. System nach einem der Ansprüche 5-7, wobei die Steuereinheit dazu konfiguriert ist, Bewegung des Benutzers auf Basis der detektierten Vibrationen zu bestimmen, wobei das Bestimmen von Bewegung des Benutzers das Bestimmen umfasst, ob eine Amplitude der detektierten Vibrationen eine vordefinierte Amplitudenschwelle überschreitet.

9. System nach einem der Ansprüche 5-8, wobei die Steuereinheit dazu konfiguriert ist, einen Schlafzustand des Benutzers auf Basis eines oder mehrerer der Herzfrequenz des Benutzers, der Atemfrequenz des Benutzers und Bewegung des Benutzers zu bestimmen.

10. System nach Anspruch 9, wobei die Steuereinheit dazu konfiguriert ist, eines oder mehrere der Folgenden durchzuführen:
in Reaktion auf das Bestimmen, dass eine oder beide der Herzfrequenz und Atemfrequenz oberhalb einer entsprechenden ersten Schwelle liegen und Variation in einer oder beiden der Herzfrequenz und Atemfrequenz unterhalb einer entsprechenden Variationsschwelle liegt, zu bestimmen, dass der Benutzer wach ist;
in Reaktion auf das Bestimmen, dass eine oder beide der Herzfrequenz und Atemfrequenz oberhalb der entsprechenden ersten Schwelle liegen und die Variation in einer oder beiden der Herzfrequenz und Atemfrequenz oberhalb der entsprechenden Variationsschwelle liegt, zu bestimmen, dass sich der Benutzer in Rapid Eye Movement-, nachstehend REM-, Schlaf befindet;
in Reaktion auf das Bestimmen, dass eine oder beide der Herzfrequenz und Atemfrequenz unterhalb der entsprechenden ersten Schwelle und oberhalb einer entsprechenden zweiten Schwelle liegen, die kleiner ist als die entsprechende erste Schwelle, zu bestimmen, dass sich der Benutzer in Leichtschlaf befindet; und
in Reaktion auf das Bestimmen, dass eine oder beide der Herzfrequenz und Atemfrequenz unterhalb der entsprechenden zweiten Schwelle liegen, zu bestimmen, dass sich der Benutzer in Tiefschlaf befindet.

11. System nach Anspruch 9 oder Anspruch 10, das weiter ein Alarmsystem umfasst, das dazu konfiguriert ist, in Reaktion auf ein Bestimmen, dass während eines Alarmzeitraums zwischen einer vordefinierten Alarmstartzeit und einer vordefinierten Alarmendzeit ein vordefinierter Schlafzustand oder eine vordefinierte Änderung des Schlafzustands erreicht wurde, einen Alarm auszugeben.

12. System nach Anspruch 11, wobei das Alarmsystem dazu konfiguriert ist, eines oder mehrere der Folgenden durchzuführen:
in Reaktion auf ein Bestimmen, dass der Benutzer während des Alarmzeitraums vom REM-Schlaf in Leichtschlaf übergegangen ist, den Alarm auszugeben;
in Reaktion auf ein Bestimmen, dass eine aktuelle Zeit innerhalb eines vordefinierten Zeitraums ab einer Alarmendzeit liegt und der Benutzer sich während des Alarmzeitraums in Leichtschlaf befindet, wobei der vordefinierte Zeitraum kürzer ist als der Alarmzeitraum, den Alarm auszugeben; und
in Reaktion darauf, dass die Alarmendzeit erreicht ist und der vordefinierte Schlafzustand oder die vordefinierte Änderung des Schlafzustands während des Alarmzeitraums nicht detektiert wurde, einen Alarm auszugeben.

## Revendications

1. Système destiné à être utilisé pour surveiller un ou plusieurs paramètres physiologiques d'un utilisateur, le système comprenant :
au moins une unité de détection (14) comprenant des capteurs de vibrations incluant un microphone configuré pour générer un signal de microphone sur la base de vibrations détectées et un capteur de pression configuré pour générer un signal de capteur de pression sur la base des vibrations détectées ;
au moins un tube élastique (12) configuré pour être intégré dans une couche d'amortissement destinée à soutenir au moins une partie de l'utilisateur, dans lequel une extrémité du au moins un tube élastique (12) est scellée et l'autre extrémité du au moins un tube élastique (12) est fermée par la au moins une unité de détection (14) de manière à former un volume qui est rempli de fluide, et qui est défini par une ou plusieurs parois internes du tube et une surface de la au moins une unité de détection (14), dans lequel le au moins un tube élastique (12) est configuré pour transmettre des vibrations d'une ou de plusieurs sections du au moins un tube élastique (12) à la au moins une unité de détection (14) afin qu'elles soient détectées par les capteurs de vibrations ; et
un dispositif de commande configuré pour déterminer une position du corps de l'utilisateur sur la base de différences de phase dans les signaux de microphone et de capteur de pression.

2. Système selon une quelconque revendication précédente, dans lequel le au moins un tube élastique (12) présente une section transversale uniforme sur toute leur longueur et/ou dans lequel le au moins un tube élastique (12) comprend une ou plusieurs indentations le long des une ou plusieurs parois internes du tube pour aider à empêcher le tube de rester collé lorsqu'il est écrasé.

3. Système selon une quelconque revendication précédente, dans lequel le au moins un tube élastique (12) comprend une ou plusieurs indentations le long des une ou plusieurs parois internes du tube pour aider à empêcher le tube de rester collé lorsqu'il est écrasé, dans lequel les une ou plusieurs indentations se présentent sous la forme d'une ou de plusieurs crêtes s'étendant le long de la longueur du tube et comprennent une pluralité d'indentations régulièrement espacées autour de la circonférence du tube.

4. Système selon la revendication 1 ou la revendication 2, dans lequel :
le dispositif de commande fait partie d'un dispositif externe et dans lequel la au moins une unité de détection (14) est configurée pour envoyer des données se rapportant aux vibrations détectées au dispositif externe destiné à être traité par le dispositif de commande ; ou
le dispositif de commande fait partie de l'unité de détection (14).

5. Système selon une quelconque revendication précédente, dans lequel le dispositif de commande est en outre configuré pour déterminer, sur la base des vibrations détectées, un ou plusieurs d'une fréquence cardiaque de l'utilisateur, d'une fréquence respiratoire de l'utilisateur et d'un mouvement de l'utilisateur.

6. Système selon la revendication 5, dans lequel le dispositif de commande est configuré pour déterminer la fréquence cardiaque de l'utilisateur, dans lequel la détermination de la fréquence cardiaque comprend :
le filtrage des vibrations détectées pour former des données de vibration filtrées sur une plage de fréquences prédéfinie ;
la détection de maxima locaux dans les données de vibration filtrées ; et
la détermination de la fréquence cardiaque sur la base des maxima locaux détectés.

7. Système selon la revendication 5 ou la revendication 6, dans lequel le dispositif de commande est configuré pour déterminer la fréquence respiratoire de l'utilisateur, dans lequel la détermination de la fréquence respiratoire comprend :
la détection de maxima locaux dans les vibrations détectées ; et
la détermination de la fréquence respiratoire sur la base des maxima locaux détectés.

8. Système selon une quelconque des revendications 5-7, dans lequel le dispositif de commande est configuré pour déterminer un mouvement de l'utilisateur sur la base des vibrations détectées, dans lequel la détermination d'un mouvement de l'utilisateur comprend la détermination si une amplitude des vibrations détectées dépasse un seuil d'amplitude prédéfini.

9. Système selon une quelconque des revendications 5-8, dans lequel le dispositif de commande est configuré pour déterminer un état de sommeil de l'utilisateur sur la base d'un ou de plusieurs de la fréquence cardiaque de l'utilisateur, de la fréquence respiratoire de l'utilisateur et d'un mouvement de l'utilisateur.

10. Système selon la revendication 9, dans lequel le dispositif de commande est configuré pour effectuer une ou plusieurs des opérations suivantes :
la détermination que l'utilisateur est éveillé à la suite de la détermination que l'une ou les deux de la fréquence cardiaque et de la fréquence respiratoire sont supérieures à un premier seuil correspondant et que la variation de l'une ou des deux de la fréquence cardiaque et de la fréquence respiratoire est inférieure à un seuil de variation correspondant ;
la détermination que l'utilisateur est dans un sommeil à mouvements oculaires rapides, ci-après dénommé REM, à la suite de la détermination que l'une ou les deux de la fréquence cardiaque et de la fréquence respiratoire sont supérieures au premier seuil correspondant et que la variation de l'une ou des deux de la fréquence cardiaque et de la fréquence respiratoire est supérieure au seuil de variation correspondant ;
la détermination que l'utilisateur est dans un sommeil léger à la suite de la détermination que l'une ou les deux de la fréquence cardiaque et de la fréquence respiratoire sont inférieures au premier seuil correspondant et supérieures à un second seuil correspondant qui est inférieur au premier seuil correspondant ; et
la détermination que l'utilisateur est dans un sommeil profond à la suite de la détermination que l'une ou les deux de la fréquence cardiaque et de la fréquence respiratoire sont inférieures au second seuil correspondant.

11. Système selon la revendication 9 ou la revendication 10, comprenant en outre un système d'alarme qui est configuré pour émettre une alarme à la suite d'une détermination qu'un état de sommeil prédéfini ou un changement prédéfini d'état de sommeil a été atteint pendant une période d'alarme entre une heure de début d'alarme prédéfinie et une heure de fin d'alarme prédéfinie.

12. Système selon la revendication 11, dans lequel le système d'alarme est configuré pour effectuer une ou plusieurs des opérations suivantes :
l'émission de l'alarme à la suite d'une détermination que l'utilisateur est passé du sommeil REM au sommeil léger pendant la période d'alarme;
l'émission de l'alarme à la suite d'une détermination qu'une heure actuelle se situe dans une période prédéfinie à partir d'une heure de fin d'alarme et que l'utilisateur est dans un sommeil léger pendant la période d'alarme, la période prédéfinie étant plus courte que la période d'alarme ; et
l'émission d'une alarme à la suite de l'atteinte de l'heure de fin d'alarme et de l'absence de détection de l'état de sommeil prédéfini ou du changement prédéfini de l'état de sommeil pendant la période d'alarme.
